# EUROPÄISCHE PATENTSCHRIFT

(11) **EP 0 992 488 B1**
(45) Veröffentlichungstag und Bekanntmachung des Hinweises auf die Patenterteilung: **02.04.2003**
(21) Anmeldenummer: 99119480.4
(22) Anmeldetag: 30.09.1999
(51) Int. Cl.: C07C 235/10, C07C 231/12, C11D 1/62, A61K 7/48, A61K 7/50, A61K 7/06

(54) **Quartäre Ammoniumverbindungen**
Quaternary ammonium compounds
Composés d'ammonium quaternaire

(30) Priorität: 09.10.1998 DE 19846538
(43) Veröffentlichungstag der Anmeldung: 12.04.2000
(73) Patentinhaber: Cognis Deutschland GmbH & Co. KG, 40589 Düsseldorf (DE)
(72) Erfinder: Llosas Bigorra, Joaquin, Dr., 08203 Sabadell (ES); Bonastre Nuria, Gilabert, Dr., 08210 Barbera del Vall (ES); Pi Subirana, Rafael, Dr., 08400 Granollers (ES); Conesa Amela, Christina, 08042 Barcelona (ES)

(56) Entgegenhaltungen:
- EP-A- 0 121 091
- EP-A- 0 253 275
- US-A- 4 874 554
- US-A- 4 923 642
- PATENT ABSTRACTS OF JAPAN vol. 012, no. 353 (C-530), 21. September 1988 (1988-09-21) & JP 63 108001 A (KYORITSU YUKI CO LTD), 12. Mai 1988 (1988-05-12)

## Beschreibung

### Gebiet der Erfindung

Die Erfindung befindet sich auf dem Gebiet der kationischen Tenside und betrifft neue quartäre Ammoniumverbindungen, Verfahren zu ihrer Herstellung sowie die Verwendung der Stoffe als Emulgatoren und Biozide.

### Stand der Technik

Quartäre Ammoniumverbindungen (QAV) stellen bekannte Tenside dar, die überwiegend durch Alkylierung von aminischen Verbindungen hergestellt werden. Vorzugsweise werden die Ausgangsstoffe mit Alkylhalogeniden oder Dialkylsulfaten umgesetzt. Quartäre Ammoniumverbindungen, wie z.B. das bekannte Distearyldimethylammoniumchlorid (DSDMAC), stellen je nach Substitutionsmuster und Alkylkettenlänge gängige Einsatzprodukte für eine Vielzahl von oberflächenaktiven Stoffen dar. QAV mit zwei langen und zwei kurzen Alkylsubstituenten am Stickstoff finden beispielsweise als Konditioniermittel in der Kosmetik sowie als Avivagemittel in Weichspülern Verwendung, während vor allem QAV mit kurzen Alkylresten oder Benzylsulbstituenten als Biozide in Reinigungs- und Desinfektionsmitteln eingesetzt werden. Eine Übersicht zu Herstellung und Verwendung von QAV findet sich beispielsweise von Bell et al. in **INFORM, 7, 992 (1996).** Ethoxylierte QAV sind aus US-A-4 874 554 bekannt.

Ein bislang ungelöstes Problem besteht jedoch darin, daß sich die bekannten quartären Ammoniumverbindungen in kaltem Wasser eher schlecht lösen und es einigen Aufwands bedarf, die meist stark getrübten Produkte zu klaren Endformulierungen zu verarbeiten. Des weiteren sind die anwendungstechnischen Eigenschaften der Produkte nicht immer zufriedenstellend.

Demzufolge hat die Aufgabe der vorliegenden Erfindung darin bestanden, neue QAV zur Verfügung zu stellen, die frei von den geschilderten Nachteilen sind, d.h. verbesserte anwendungstechnische Ei-genschaften aufweisen und sich insbesondere auch in kaltem Wasser rasch und trübungsfrei lösen.

### Beschreibung der Erfindung

Gegenstand der Erfindung sind quartäre Ammoniumverbindungen der Formel **(I)**, 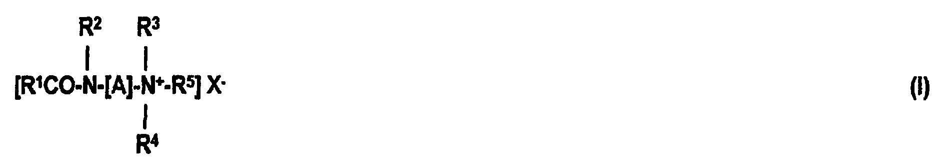 in der R¹CO für einen gesättigten und/oder ungesättigten ethoxylierten Hydroxyacylrest mit 16 bis 22, vorzugsweise 18 Kohlenstoffatomen sowie 1 bis 50 Oxyethyleneinheiten, A für einen linearen oder verzweigten Alkylenrest mit 1 bis 6 Kohlenstoffatomen, R², R³ und R⁴ unabhängig voneinander für Wasserstoff oder eine Alkylgruppe mit 1 bis 4 Kohlenstoffatomen, R⁵ für einen Alkylrest mit 1 bis 4 Kohlenstoffatomen oder einen Benzylrest und X für Halogen, Alkylsulfat oder Alkylphosphat steht.

Überraschenderweise wurde gefunden, daß die Anlagerung von Ethylenoxid an die OH-Gruppe von Ricinolsäure bzw. 12-Hydroxystearinsäure zu quartären Ammoniumverbindungen führt, die nicht nur verbesserte anwendungstechnische Eigenschaften aufweisen, sondern sich auch in kaltem Wasser klar lösen und dann zu trübungsfreien Endformulierungen weiterverarbeitet werden können.

### Herstellverfahren

Ein weiterer Gegenstand der Erfindung betrifft Verfahren zur Herstellung von quartären Ammoniumverbindungen der Formel **(I)**, 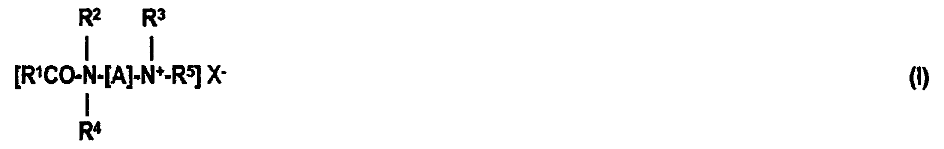 in der R¹CO für einen gesättigten und/oder ungesättigten ethoxylierten Hydroxyacylrest mit 16 bis 22, vorzugsweise 18 Kohlenstoffatomen sowie 1 bis 50 Oxyethyleneinheiten, A für einen linearen oder verzweigten Alkylenrest mit 1 bis 6 Kohlenstoffatomen, R², R³ und R⁴ unabhängig voneinander für Wasserstoff oder eine Alkylgruppe mit 1 bis 4 Kohlenstoffatomen, R⁵ für einen Alkylrest mit 1 bis 4 Kohlenstoffatomen oder einen Benzylrest und X für Halogen, Alkylsulfat oder Alkylphosphat steht, bei dem man
(a) Ricinusöl undloder gehärtetes Ricinusöl mit - bezogen auf die Hydroxylgruppen im Acylrest - 1 bis 50 Mol Ethylenoxid ethoxyliert,
(b) die resultierende Ethoxylate mit Diaminen umsetzt und
(c) die resultierenden Amidoamine mit Benzylhalogeniden bzw. Alkylhalogeniden, -sulfaten oder - phosphaten mit 1 bis 4 Kohlenstoffatomen quaterniert.

Alternativ ist es auch möglich, zunächst das Triglycerid in das Amidoamin zu überführen und dieses dann zu ethoxylieren, ehe anschließend die Quaternierung mit den Alkylierungsmitteln erfolgt. Dementsprechend betrifft ein weiterer Gegenstand der Erfindung ein Verfahren zur Herstellung von quartären Ammoniumverbindungen der oben genannten Formel **(I),** bei dem man
(a) Ricinusöl und/oder gehärtetes Ricinusöl mit Diaminen umsetzt,
(b) die resultierende Amidoamine mit - bezogen auf die Hydroxylgruppen im Acylrest - 1 bis 50 Mol Ethylenoxid ethoxyliert und
(c) die resultierenden ethoxylierten Amidoamine mit Benzylhalogeniden bzw. Alkylhalogeniden, -sul-faten oder -phosphaten mit 1 bis 4 Kohlenstoffatomen quaterniert.

Schließlich ist es auch möglich, zunächst aus dem Triglycerid das Amidoamin und aus diesem die quartären Ammoniumverbindungen herzustellen, ehe dann abschließend Ethylenoxid an die Hydroxyl-gruppe des Acylrestes angelagert wird. Dementsprechend betrifft ein weiterer Gegenstand der Erfindung ein drittes Verfahren zur Herstellung von quartären Ammoniumverbindungen der Formel **(I)**, bei dem man
(a) Ricinusöl und/oder gehärtetes Ricinusöl mit Diaminen umsetzt,
(b) die resultierende Amidoamine mit Benzylhalogeniden bzw. Alkylhalogeniden, -sulfaten oder -phosphaten mit 1 bis 4 Kohlenstoffatomen quaterniert, und
(c) die resultierenden QAV mit - bezogen auf die Hydroxylgruppen im Acylrest - 1 bis 50 Mol Ethylenoxid ethoxyliert.

### Ethoxylierung

Wie oben erläutert kann die Ethoxylierung auch auf der Stufe der Amidoamine oder der quartären Am-moniumverbindungen durchgeführt werden. Vorzugsweise legt man jedoch die Triglyceride zusammen mit 0,5 bis 2,5 Gew.-% Katalysator (z.B. Natriummethylat oder calcinierter Hydrotalcit) in einen Rührautoklaven vor, evakuiert und gibt bei Temperaturen im Bereich von 120 bis 180 °C innerhalb von 1 bis 2 h die gewünschte Menge Ethylenoxid, vorzugsweise 5 bis 10 Mol Ethylenoxid - bezogen auf die Hy-droxylgruppen im Acylrest - auf, wobei der autogene Druck bis auf 5 bar ansteigen kann. Anschließend läßt man noch etwa 30 min nachreagieren, kühlt den Reaktor ab, entspannt und neutralisiert den basischen Katalysator z.B. durch Zugabe von Milchsäure oder Phosphorsäure. Gegebenenfalls werden unlösliche Katalysatoren über eine Filterpresse abgetrennt

### Amidierung

Die Amidierung der Triglyceride kann in an sich bekannter Weise durchgeführt werden. Vorzugsweise gelangen hierzu Diamine der Formel **(II)** zum Einsatz, 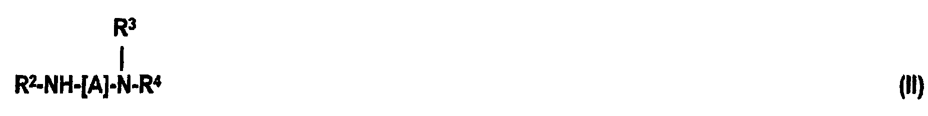 in der A für einen linearen oder verzweigten Alkylenrest mit 1 bis 6 Kohlenstoffatomen und R², R³ und R⁴ unabhängig voneinander für Wasserstoff oder eine Alkylgruppe mit 1 bis 4 Kohlenstoffatomen stehen. Neben Propylendiamin, Butylendiamin, Pentylendiamin und Hexylendiamin wird vorzugsweise Dimethylaminopropylamin (DAPA) eingesetzt. Dazu empfiehlt es sich, das Diamin - bezogen auf die für die Amidierung zur Verfügung stehenden Carboxylgruppen im Triglycerid - im geringen stöchiometrischen Überschuß, also im molaren Verhältnis 1:1,05 bis 1 : 1,2 einzusetzen. Die Amidierung kann in Gegenwart von für diesen Zweck üblichen alkalischen Katalysatoren, wie z.B. Natriumhydroxid, Kaliumhydroxid oder Natriummethylat durchgeführt werden; die Katalysatorkonzentration liegt in der Regel bei 0,1 bis 2 Gew.-% - bezogen auf die Einsatzstoffe. Die Mitverwendung von geringen Mengen Alkaliborhydraten oder unterphosphoriger Säure als Co-Katalysatoren empfiehlt sich, wenn es darum geht, möglichst hellfarbige Produkte herzustellen. Die Reaktionstemperatur beträgt 100 bis 150 und vorzugsweise 120 bis 140°C. Falls gewünscht, kann unter Stickstoffabdeckung gearbeitet werden. Nach Abschluß der Amidierung kann nicht umgesetztes Diamin, gegebenenfalls zusammen mit dem freigesetzten Glycerin, im Feinvakuum abdestilliert werden. Wie schon eingangs erläutert, kann die Amidierung sowohl mit dem ethoxylierten als auch dem nicht ethoxylierten Triglycerid durchgeführt werden.

### Quaternierung

Zur Überführung der vorzugsweise bereits ethoxylierten Amidoamine in die quartären Ammoniumverbindungen erfolgt eine Kondensation der Aminofunktion mit einem Benzylhalogenid, einem C₁-C₄ Alkylhalogenid, einem C₁-C₄ Alkylsulfat oder C₁-C₄-Alkylphosphat. Vorzugsweise werden Benzylchlorid, Methylchlorid oder Dimethylsulfat eingesetzt. Wie schon die Ethoxylierung und die Amidierung erfolgt auch die Quaternierung in an sich bekannter Weise, d.h. das Amidoamin wird in Substanz, in wäßriger oder alkoholischer Lösung mit dem Alkylierungsmittel bei Temperaturen im Bereich von 70 bis 100°C kondensiert. Die Reaktionspartner werden dabei in annähernd stöchiometrischem Verhältnis, d.h. 1 : 0,95 bis 1 : 1,05 eingesetzt. Es empfiehlt sich, während der Kondensation den pH-Wert durch Zudosieren einer wäßrigen Base im alkalischen Bereich zu halten und erst das Endprodukt durch Zugabe von Mineralsäure leicht sauer einzustellen.

### Gewerbliche Anwendbarkeit

Die neuen quartären Ammoniumverbindungen, insbesondere die methylquarternierten Salze, weisen ausgezeichnete emulgierende und konditionierende Eigenschaften auf, sind auch in kaltem Wasser klarlöslich und lassen sich zu trübungsfreien Endprodukten formulieren. Ein weiterer Gegenstand der Erfindung betrifft daher die Verwendung der erfindungsgemäßen quartären Ammoniumverbindungen als kationische Emulgatoren zur Herstellung von kosmetischen- und/oder pharmazeutischen Zubereitungen, in denen sie in Mengen von 0,5 bis 50, vorzugsweise 1 bis 20 und insbesondere 5 bis 15 Gew.-% - bezogen auf die Mittel - enthalten sein können. Darüber hinaus weisen insbesondere die benzylquaternierten Salze bakteriostatische Eigenschaften auf, so daß ein weiterer Gegenstand der Erfindung die Verwendung der erfindungsgemäßen quartären Ammoniumverbindungen als Biozide zur Herstellung von Reinigungs- und Desinfektionsmittel betrifft, in denen sie ebenfalls in Mengen von 0,5 bis 50, vorzugsweise 1 bis 20 und insbesondere 5 bis 15 Gew.-%-bezogen auf die Mittel - enthalten sein können.

### Kosmetische und/oder pharmazeutische Zubereitungen

Die die neuen quartären Ammoniumverbindungen enthaltenden Zubereitungen, wie beispielsweise Haarshampoos, Haarlotionen, Haarkuren, Conditioner, Schaumbäder, Cremes, Lotionen, Salben und dergleichen, können ferner als weitere Hilfs- und Zusatzstoffe milde Tenside, Ölkörper, Emulgatoren, Überfettungsmittel, Perlglanzwachse, Stabilisatoren, Konsistenzgeber, Verdickungsmittel, Polymere, Siliconverbindungen, biogene Wirkstoffe, Deowirkstoffe, Antischuppenmittel, Filmbildner, Konservierungsmittel, Hydrotrope, Solubilisatoren, UV-Lichtschutzfaktoren, Antioxidantien, Insektenrepellentien, Selbstbräuner, Parfümöle, Farbstoffe und dergleichen enthalten.

Typische Beispiele für geeignete milde, d.h. besonders hautverträgliche **Tenside** sind Fettalkoholpoly-glycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) undloder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen undloder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlen-stoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe in Betracht.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipenta-erythrit, Zuckeralkohole (z.B. Sorbit), Alkylglucoside (z.B. Methylglucosid, Butylglucosid, Lauryl-glucosid) sowie Polyglucoside (z.B. Cellulose);
(9) Mono-, Di- und Trialkylphosphate sowie Mono-, Di- und/oder Tri-PEG-alkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin oder Polyglycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 2024051** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylamino-propyl-N,N-dimethylammoniumglycinate, beispielsweise das Kokosacylaminopropyldimethylammonium-glycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquatemierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden undloder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethyl-cellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologen-verteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhält-lich ist, kationische Stärke, Copolymere von Diallylammoniumsalzen und Acrylamiden, quaternierte Vinylpyrrolidon/Vinyl-imidazol-Polymere, wie z.B. Luviquat® (BASF), Kondensationsprodukte von Poly-glycolen und Aminen, quaternierte Kollagenpolypeptide, wie beispielsweise Lauryldimonium hydroxy-propyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR-A 2252840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/ lsobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Eine detaillierte Übersicht über geeignete flüchtige Silicone findet sich zudem von Todd et al. in **Cosm.Toil. 91, 27(1976).**

Typische Beispiele für **Fette** sind Glyceride, als **Wachse** kommen u.a. Bienenwachs, Camaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säu-ren, Aminosäuren, Ceramide, Pseudoceramide, essentielle Öle, Pflanzenextrakte und Vitaminkomplexe zu verstehen.

Als **Deowirkstoffe** kommen z.B. Antiperspirantien wie etwa Aluminiumchlorhydate in Frage. Hierbei handelt es sich um farblose, hygroskopische Kristalle, die an der Luft leicht zerfließen und beim Eindampfen wäßriger Aluminiumchloridlösungen anfallen. Aluminiumchlorhydrat wird zur Herstellung von schweißhemmenden und desodorierenden Zubereitungen eingesetzt und wirkt wahrscheinlich über den partiellen Verschluß der Schweißdrüsen durch Eiweiß- und/oder Polysaccharidfällung [vgl. **J.Soc.Cosm.Chem. 24, 281 (1973)]**. Unter der Marke Locron® der Hoechst AG, Frankfurt/FRG, befindet beispielsweise sich ein Aluminiumchlorhydrat im Handel, das der Formel [Al₂(OH)₅Cl]*2,5 H₂O entspricht und dessen Einsatz besonders bevorzugt ist [vgl. **J.Pharm.Pharmacol. 26, 531 (1975)]**. Neben den Chlorhydraten können auch Aluminiumhydroxylactate sowie saure Aluminium/Zirko-niumsalze eingesetzt werden. Als weitere Deowirkstoffe können Esteraseinhibitoren zugesetzt werden. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, daß dadurch die Enzyme inhibiert werden. Weitere Stoffe, die als Esteraseinhibitoren in Betracht kommen, sind Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester. Antibakterielle Wirkstoffe, die die Keimflora beeinflussen und schweißzersetzende Bakterien abtöten bzw. in ihrem Wachstum hemmen, können ebenfalls in den Stiftzubereitungen enthalten sein. Beispiele hierfür sind Chitosan, Phenoxyethanol und Chlorhexidingluconat. Besonders wirkungsvoll hat sich auch 5-Chlor-2-(2,4-dichlorphen-oxy)-phenol erwiesen, das unter der Marke Irgasan® von der Ciba-Geigy, Basel/CH vertrieben wird.

Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylben-zyliden)campher wie in der **EP-B1 0693471** beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäure-propylester, 4-Methoxyzimtsäureisoamylester 2-Cyano-3,3-phenylzimtsäure-2-ehhylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-meth-oxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon, wie in der **EP-A1 0818450** beschrieben;
- Propan-1,3-dione, wie z.B. 1-(4-tert.Butylphenyl)-3-(4'methoxyphenyl)propan-1,3-dion;
- Ketotricydo(5.2.1.0)decan-Derivate, wie in der **EP-B1 0694521** beschrieben.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsul-fonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoyl-methan (Parsol 1789), oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Weitere geeignete UV-Lichtschutzfilter sind der Übersicht von P.Finkel in **SÖFW-Journal 122, 543 (1996)** zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Auro-thioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearyl-thiodipropionat, Thiodipropionsäure und deren Derivate (Ester, Ether, Peptide, Lipide, Nukleotide, Nuk-leoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Deri-vate, α-Glycosylrutin, Ferulasäure, Furfurylidenglucitol, Carnosin, Butylhydroxytoluol, Butylhydroxy-anisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner **Hydrotrope**, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-touluamid, 1,2-Pentandiol oder Insect repellent 3535 in Frage, als **Selbstbräuner** eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Iso-methylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwen-det, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiver-öl, Olibanöl, Galbanumöl, Labolanumöl und Lavandinöl. Vorzugsweise werden Bergamotteöl, Dihydro-myrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

**Herstellbeispiel H1.** In einem 2-I-Dreihalskolben mit Rührer, Rückflußkühler und Destillationsaufsatz wurden 1084 g (0,86 mol) eines Kondensationsproduktes von 8 Mol Ethylenoxid an Ricinusöl zusammen mit 0,45 g Natriumborhydrat, 342 g (3,35 mol) Dimethylaminpropylamin (DAPA) und 28 g einer 33 Gew.-%igen Lösung von Natriummethylat in Methanol vorgelegt. Die Mischung wurde über 2 h auf 130°C erhitzt und anschließend nicht umgesetztes DAPA destillativ entfernt (150°C, 40 mbar). Das resultierende Amidoamin wies einen Estergehalt von weniger als 0,5 Gew.-% auf. 300 g (0,65 mol) des Amidoamins wurden in eine zweite Rührapparatur überführt, in 93 ml Wasser gelöst und auf 50°C erwärmt. Anschließend wurden über einen Tropftrichter 82 g (0,64 mol) Benzylchlorid zugegeben und weitere 6 h bei 60°C gerührt. Es wurde eine gelbliche Flüssigkeit mit einem Feststoffgehalt von 80 Gew.-% erhalten.

**Herstellbeispiel H2.** In einem 2-I-Dreihalskolben mit Rührer, Rückflußkühler und Destillationsaufsatz wurden 1084 g (0,86 mol) eines Kondensationsproduktes von 8 Mol Ethylenoxid an Ricinusöl zusammen mit 0,45 g Natriumborhydrat, 342 g (3,35 mol) Dimethylaminpropylamin (DAPA) und 28 g einer 33 Gew.-%igen Lösung von Natriummethylat in Methanol vorgelegt. Die Mischung wurde über 2 h auf 130°C erhitzt und anschließend nicht umgesetztes DAPA destillativ entfernt (150°C, 40 mbar). Das resultierende Amidoamin wies einen Estergehalt von weniger als 0,5 Gew.-% auf. 74 g (0,16 mol) des Amidoamins wurden in eine zweite Rührapparatur überführt, mit 19 g (0,15 mol) Dimethylsulfat versetzt und weitere 4 h bei 65°C gerührt. Das resultierende, leicht gelb gefärbte Quaternierungsprodukt war bei Raumtemperatur flüssig.

Die nachfolgende Tabelle 1 zeigt eine Reihe von Beispielen zur Formulierung der erfindungsgemäßen quartären Ammoniumverbindungen in kosmetischen Zubereitungen.

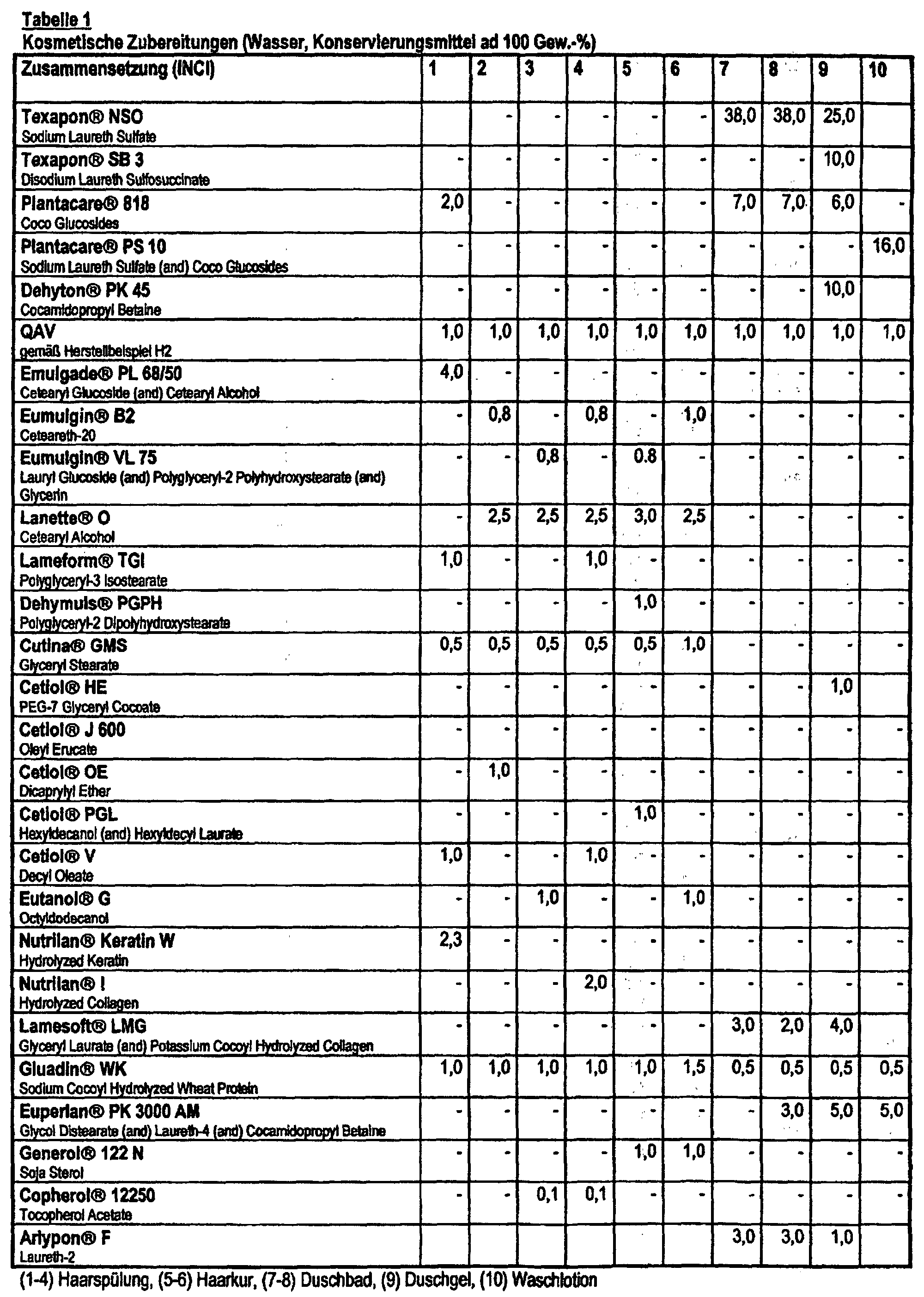

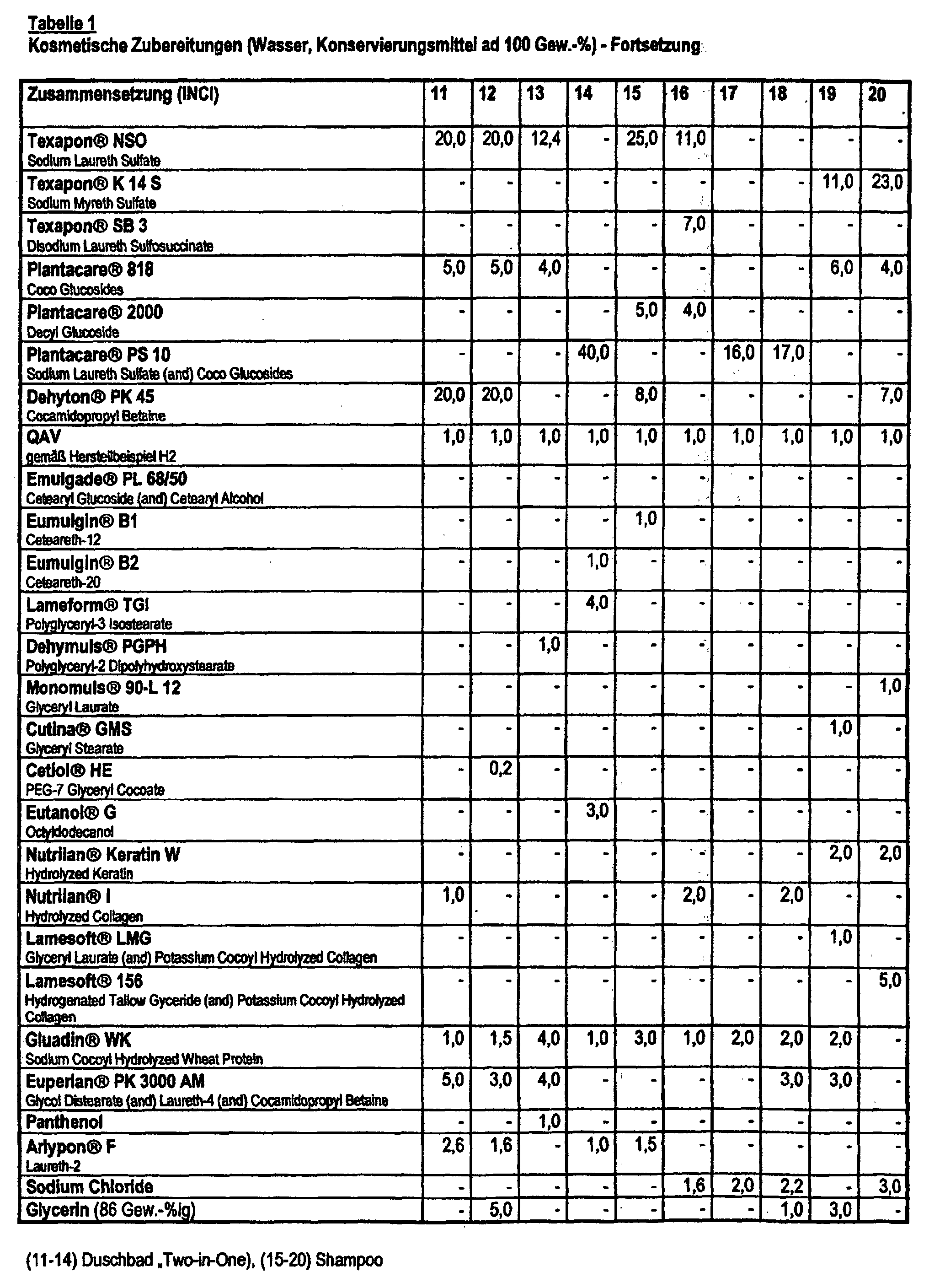

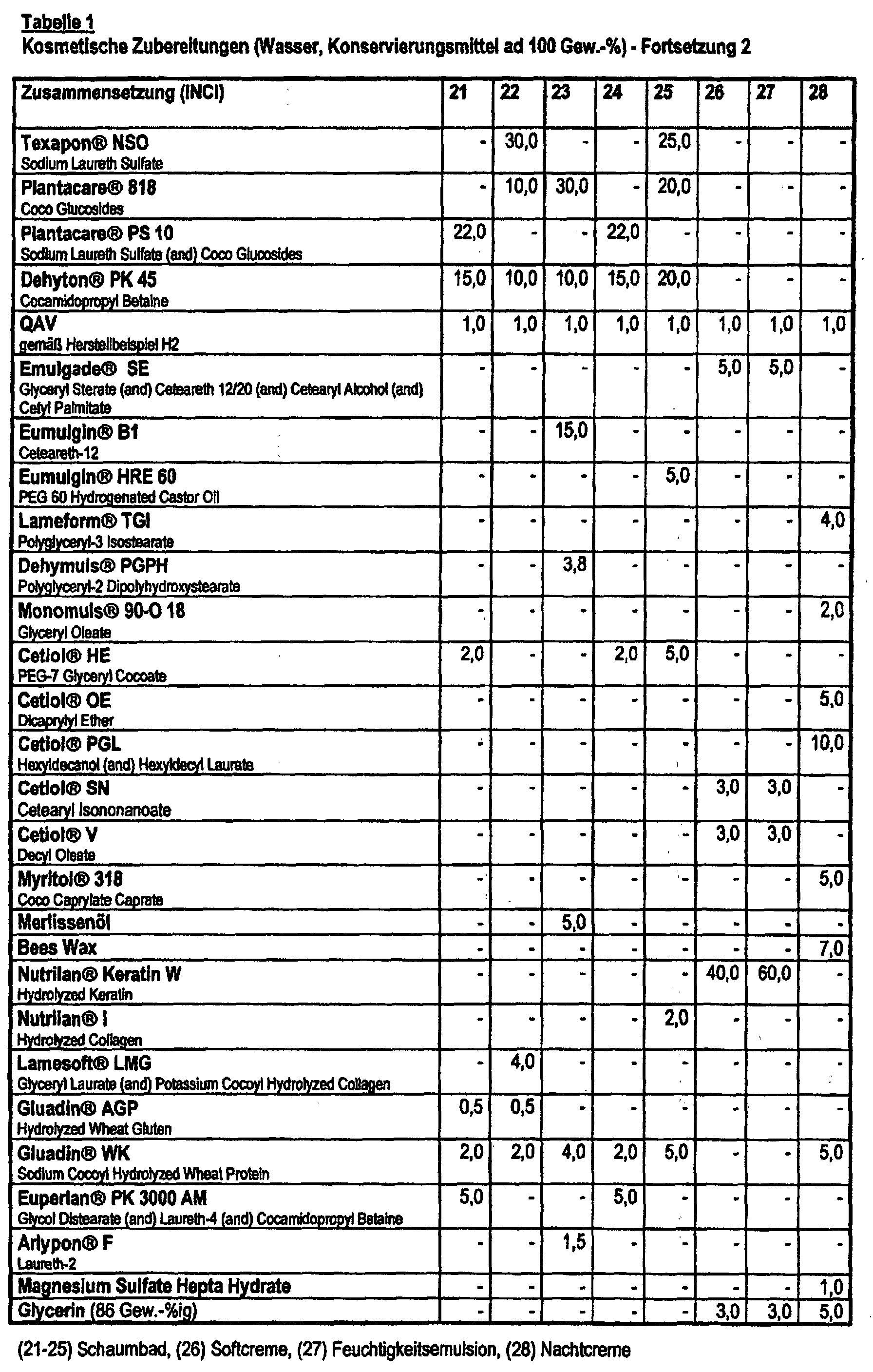

## Patentansprüche

1. Quartäre Ammoniumverbindungen (QAV) der Formel **(I)**, 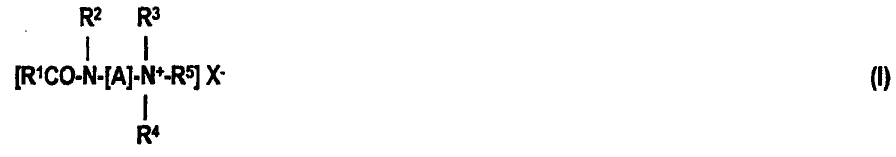 in der R¹CO für einen gesättigten und/oder ungesättigten ethoxylierten Hydroxyacylrest mit 16 bis 22 Kohlenstoffatomen sowie 1 bis 50 Oxyethyleneinheiten, A für einen linearen oder verzweigten Alkylenrest mit 1 bis 6 Kohlenstoffatomen, R², R³ und R⁴ unabhängig voneinander für Wasserstoff oder eine Alkylgruppe mit 1 bis 4 Kohlenstoffatomen, R⁵ für einen Alkylrest mit 1 bis 4 Kohlenstoffatomen oder einen Benzylrest und X für Halogen, Alkylsulfat oder Alkylphosphat steht.

2. Verfahren zur Herstellung von quartären Ammoniumverbindungen der Formel **(I)**, 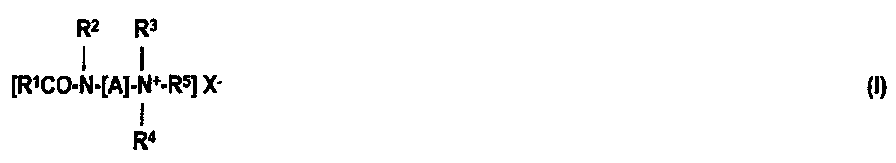 in der R¹CO für einen gesättigten und/oder ungesättigten ethoxylierten Hydroxyacylrest mit 16 bis 22 Kohlenstoffatomen sowie 1 bis 50 Oxyethyleneinheiten, A für einen linearen oder verzweigten Alkylenrest mit 1 bis 6 Kohlenstoffatomen, R², R³ und R⁴ unabhängig voneinander für Wasserstoff oder eine Alkylgruppe mit 1 bis 4 Kohlenstoffatomen, R⁵ für einen Alkylrest mit 1 bis 4 Kohlenstoffatomen oder einen Benzylrest und X für Halogen, Alkylsulfat oder Alkylphosphat steht, bei dem man
(a) Ricinusöl und/oder gehärtetes Ricinusöl mit - bezogen auf die Hydroxylgruppen im Acylrest - 1 bis 50 Mol Ethylenoxid ethoxyliert,
(b) die resultierende Ethoxylate mit Diaminen umsetzt und
(c) die resultierenden Amidoamine mit Benzylhalogeniden bzw. Alkylhalogeniden, -sulfaten oder -phosphaten mit 1 bis 4 Kohlenstoffatomen quaterniert.

3. Verfahren zur Herstellung von quartären Ammoniumverbindungen der Formel **(I)**, 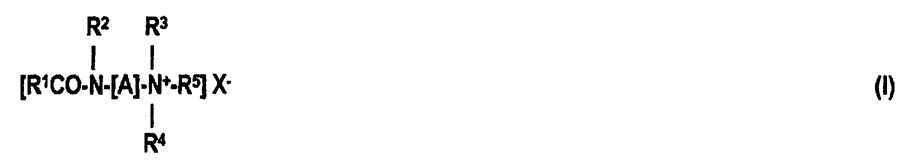 in der R¹CO für einen gesättigten und/oder ungesättigten ethoxylierten Hydroxyacylrest mit 16 bis 22 Kohlenstoffatomen sowie 1 bis 50 Oxyethyleneinheiten, A für einen linearen oder verzweigten Alkylenrest mit 1 bis 6 Kohlenstoffatomen, R², R³ und R⁴ unabhängig voneinander für Wasserstoff oder eine Alkylgruppe mit 1 bis 4 Kohlenstoffatomen, R⁵ für einen Alkylrest mit 1 bis 4 Kohlenstoffatomen oder einen Benzylrest und X für Halogen, Alkylsulfat oder Alkylphosphat steht, bei dem man
(a) Ricinusöl und/oder gehärtetes Ricinusöl mit Diaminen umsetzt,
(b) die resultierende Amidoamine mit - bezogen auf die Hydroxylgruppen im Acylrest - 1 bis 50 Mol Ethylenoxid ethoxyliert und
(c) die resultierenden ethoxylierten Amidoamine mit Benzylhalogeniden bzw. Alkylhalogeniden, -sulfaten oder-phosphaten mit 1 bis 4 Kohlenstoffatomen quaterniert.

4. Verfahren zur Herstellung von quartären Ammoniumverbindungen der Formel **(I)**, 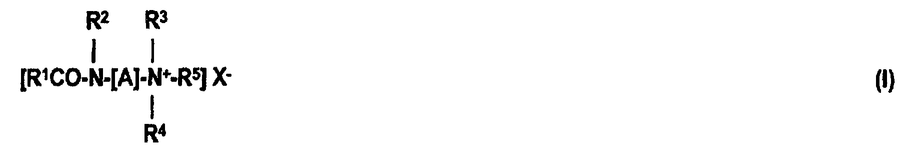 in der R¹CO für einen gesättigten und/oder ungesättigten ethoxylierten Hydroxyacylrest mit 16 bis 22 Kohlenstoffatomen sowie 1 bis 50 Oxyethyleneinheiten, A für einen linearen oder verzweigten Alkylenrest mit 1 bis 6 Kohlenstoffatomen, R², R³ und R⁴ unabhängig voneinander für Wasserstoff oder eine Alkylgruppe mit 1 bis 4 Kohlenstoffatomen, R⁵ für einen Alkylrest mit 1 bis 4 Kohlenstoffatomen oder einen Benzylrest und X für Halogen, Alkylsulfat oder Alkylphosphat steht, bei dem man
(a) Ricinusöl und/oder gehärtetes Ricinusöl mit Diaminen umsetzt,
(b) die resultierende Amidoamine mit Benzylhalogeniden bzw. Alkylhalogeniden, -sulfaten oder -phosphaten mit 1 bis 4 Kohlenstoffatomen quaterniert, und
(c) die resultierenden QAV mit - bezogen auf die Hydroxylgruppen im Acylrest - 1 bis 50 Mol Ethylenoxid ethoxyliert.

5. Verfahren nach mindestens einem der Ansprüche 2 bis 4, **dadurch gekennzeichnet, daß** man die Ethoxylierung mit 5 bis 10 Mol Ethylenoxid - bezogen auf die Hydroxylgruppen im Acylrest - durchführt.

6. Verfahren nach mindestens einem der Ansprüche 2 bis 5, **dadurch gekennzeichnet, daß** man Diamine der Formel **(II)** einsetzt, 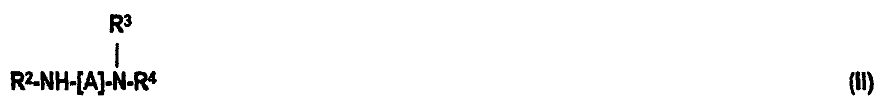 in der A für einen linearen oder verzweigten Alkylenrest mit 1 bis 6 Kohlenstoffatomen und R², R³ und R⁴ unabhängig voneinander für Wasserstoff oder eine Alkylgruppe mit 1 bis 4 Kohlenstoffatomen stehen.

7. Verfahren nach Anspruch 6, **dadurch gekennzeichnet, daß** man Dimethylaminopropylamin einsetzt.

8. Verfahren nach mindestens einem der Ansprüche 2 bis 7, **dadurch gekennzeichnet, daß** man zur Quaternierung Benzylchlorid, Methylchlorid oder Dimethylsulfat einsetzt.

9. Verwendung der quartären Ammoniumverbindungen nach Anspruch 1 als kationische Emulgatoren zur Herstellung von kosmetischen- und/oder pharmazeutischen Zubereitungen.

10. Verwendung der quartären Ammoniumverbindungen nach Anspruch 1 als Biozide zur Herstellung von Reinigungs- und Desinfektionsmitteln.

## Claims

1. Quaternary ammonium compounds (QUATS) corresponding to formula **(I)**: 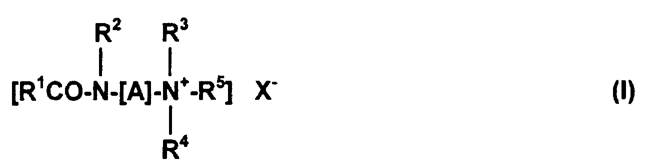 in which R¹CO is a saturated and/or unsaturated ethoxylated hydroxyacyl group containing 16 to 22 carbon atoms and 1 to 50 oxyethylene units, A is a linear or branched alkylene group containing 1 to 6 carbon atoms, R², R³ and R⁴ independently of one another represent hydrogen or a C₁₋₄ alkyl group, R⁵ is a C₁₋₄ alkyl group or a benzyl group and X is halogen, alkyl sulfate or alkyl phosphate.

2. A process for the production of quaternary ammonium compounds corresponding to formula **(I)**: 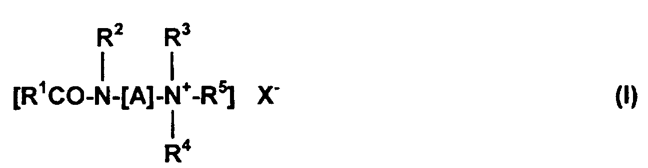 in which R¹CO is a saturated and/or unsaturated ethoxylated hydroxyacyl group containing 16 to 22 carbon atoms and 1 to 50 oxyethylene units, A is a linear or branched alkylene group containing 1 to 6 carbon atoms, R², R³ and R⁴ independently of one another represent hydrogen or a C₁₋₄ alkyl group, R⁵ is a C₁₋₄ alkyl group or a benzyl group and X is halogen, alkyl sulfate or alkyl phosphate,
**characterized in that**
(a) castor oil and/or hydrogenated castor oil is ethoxylated with 1 to 50 mol of ethylene oxide, based on the hydroxyl groups in the acyl group,
(b) the resulting ethoxylates are reacted with diamines and
(c) the resulting amidoamines are quaternized with benzyl halides or alkyl halides, sulfates or phosphates containing 1 to 4 carbon atoms.

3. A process for the production of quaternary ammonium compounds corresponding to formula **(I)**: 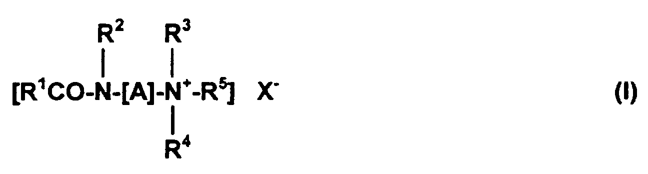 in which R¹CO is a saturated and/or unsaturated ethoxylated hydroxyacyl group containing 16 to 22 carbon atoms and 1 to 50 oxyethylene units, A is a linear or branched alkylene group containing 1 to 6 carbon atoms, R², R³ and R⁴ independently of one another represent hydrogen or a C₁₋₄ alkyl group, R⁵ is a C₁₋₄ alkyl group or a benzyl group and X is halogen, alkyl sulfate or alkyl phosphate,
**characterized in that**
(a) castor oil and/or hydrogenated castor oil is reacted with diamines,
(b) the resulting amidoamines are ethoxylated with 1 to 50 mol of ethylene oxide, based on the hydroxyl groups in the acyl group, and
(c) the resulting ethoxylated amidoamines are quaternized with benzyl halides or alkyl halides, sulfates or phosphates containing 1 to 4 carbon atoms.

4. A process for the production of quaternary ammonium compounds corresponding to formula **(I)**: 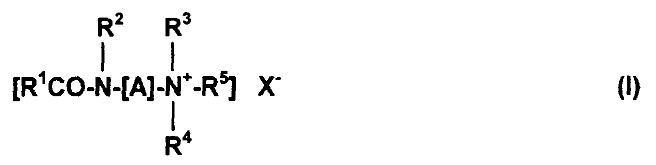 in which R¹CO is a saturated and/or unsaturated ethoxylated hydroxyacyl group containing 16 to 22 carbon atoms and 1 to 50 oxyethylene units, A is a linear or branched alkylene group containing 1 to 6 carbon atoms, R², R³ and R⁴ independently of one another represent hydrogen or a C₁₋₄ alkyl group, R⁵ is a C₁₋₄ alkyl group or a benzyl group and X is halogen, alkyl sulfate or alkyl phosphate,
**characterized in that**
(a) castor oil and/or hydrogenated castor oil is reacted with diamines,
(b) the resulting amidoamines are quaternized with benzyl halides or alkyl halides, sulfates or phosphates containing 1 to 4 carbon atoms and
(c) the resulting QUATS are ethoxylated with 1 to 50 mol of ethylene oxide, based on the hydroxyl groups in the acyl group.

5. A process as claimed in at least one of claims 2 to 4, **characterized in that** the ethoxylation is carried out with 5 to 10 mol ethylene oxide, based on the hydroxyl groups in the acyl group.

6. A process as claimed in at least one of claims 2 to 5, **characterized in that** diamines corresponding to formula **(II)**: 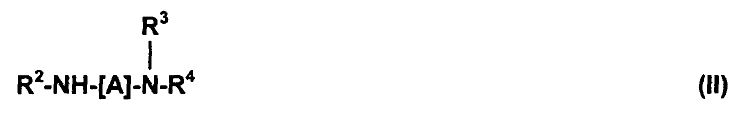 in which A is a linear or branched alkylene group containing 1 to 6 carbon atoms and R², R³ and R⁴ independently of one another represent hydrogen or a C₁₋₄ alkyl group,
are used.

7. A process as claimed in claim 6, **characterized in that** dimethylaminopropylamine is used.

8. A process as claimed in at least one of claims 2 to 7, **characterized in that** benzyl chloride, methyl chloride or dimethyl sulfate is used for the quaternization.

9. The use of the quaternary ammonium compounds claimed in claim 1 as cationic emulsifiers for the production of cosmetic and/or pharmaceutical preparations.

10. The use of the quaternary ammonium compounds claimed in claim 1 as biocides for the production of detergents.

## Revendications

1. Composés d'ammonium quaternaires (QAV) de formule (I) 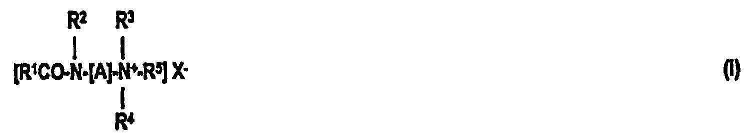 dans laquelle R¹CO représente un reste hydroxyacyle éthoxylé, saturé et/ou non saturé, ayant de 16 à 22 atomes de carbone ainsi que de 1 à 50 éléments oxyéthylène, A représente un reste alkylène linéaire ou ramifié ayant de 1 à 6 atomes de carbone, R², R³ et R⁴ indépendamment l'un de l'autre représentent de l'hydrogène ou un groupe alkyle ayant de 1 à 4 atomes de carbone, R⁵ représente un reste alkyle ayant de 1 à 4 atomes de carbone ou un reste benzyle et X représente un halogène, un alkylsulfate ou un alkylphosphate.

2. Procédé de préparation de composés d'ammonium quaternaires de formule (I) 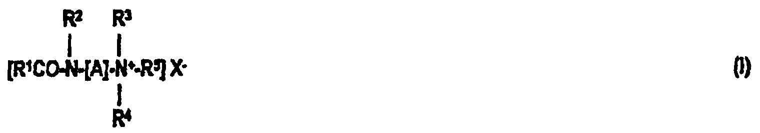 dans laquelle R¹CO représente un reste hydroxyacyle éthoxylé saturé et/ou non saturé ayant de 16 à 22 atomes de carbone ainsi que de 1 à 50 éléments oxyéthylène, A représente un reste alkylène linéaire ou ramifié ayant de 1 à 6 atomes de carbone, R², R³ et R⁴, indépendamment l'un de l'autre, représentent de l'hydrogène ou un groupe alkyle ayant de 1 à 4 atomes de carbone, R⁵ représente un reste alkyle ayant de 1 à 4 atomes de carbone ou un reste benzyle et X représente un halogène, un alkylsulfate ou un alkylphosphate
dans lequel on :
(a) éthoxyle de l'huile de ricin et/ou de l'huile de ricin durcie avec - rapporté aux groupes hydroxyles dans le reste acyle - 1 à 50 mol d'oxyde d'éthylène,
(b) fait réagir l'éthoxylate résultant avec des diamines et
(c) quaternise les amidoamines résultants avec les halogénures de benzyle ou avec de alkylhalogénures, des alkylsulfates ou des alkylphosphates ayant de 1 à 4 atomes de carbone.

3. Procédé de production de composés d'ammonium quaternaires de formule (I) 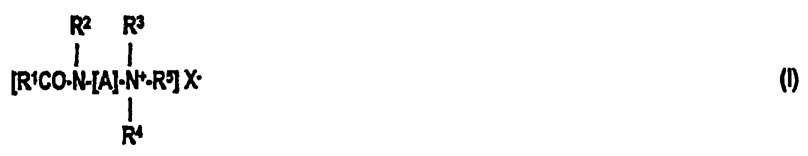 dans laquelle
R¹CO représente un reste hydroxyacyle éthoxylé saturé et/ou non saturé, ayant de 16 à 22 atomes de carbone, ainsi que de 1 à 50 éléments oxyéthylène, A représente un reste alkylène linéaire ou ramifié ayant de 1 à 6 atomes de carbone, R², R³ et R⁴ indépendamment l'un de l'autre représentent de l'hydrogène ou un groupe alkyle ayant de 1 à 4 atomes de carbone, R⁵ représente un reste alkyle ayant de 1 à 4 atomes de carbone ou un reste benzyle, et X représente un halogène, un alkyle sulfate ou un alkyl phosphate
dans lequel on
(a) fait réagir de l'huile de ricin ou de l'huile de ricin durcie avec des diamines
(b) éthoxyle l'amidoamine résultante avec - rapporté aux groupes hydroxyle dans le reste acyle - de 1 à 50 mol d'oxyde d'éthylène et
(c) quaternise l'amidoamine éthoxylée résultante avec des halogénures de benzyle, des alkylhalogénures, -sulfates ou -phosphates ayant de 1 à 4 atomes de carbone.

4. Procédé de préparation de composés d'ammonium quaternaires de formule (I) 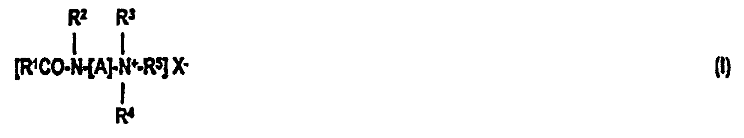 dans laquelle R¹CO représente un reste hydroxyacyle éthoxylé saturé et/ou non saturé ayant de 16 à 22 atomes de carbone ainsi que de 1 à 50 éléments oxyéthylène, A représente un reste alkylène linéaire ou ramifié ayant de 1 à 6 atomes de carbone, R², R³ et R⁴ indépendamment l'un de l'autre représentent de l'hydrogène, ou un groupe alkyle ayant de 1 à 4 atomes de carbone, R⁵ représente un reste alkyle ayant de 1 à 4 atomes de carbone ou un reste benzyle et X représente un halogène, un alkylsulfate ou un alkylphosphate,
dans lequel on :
(a) fait réagir de l'huile de ricin et/ou de l'huile de ricin durcie avec des diamines,
(b) quaternise les amidoamines résultantes avec des halogénures de benzyle ou des alkylhalogénures, des alkylsulfates ou des alkylphosphates ayant de 1 à 4 atomes de carbone et
(c) éthoxyle les QAV résultants avec - rapporté aux groupes hydroxyle dans le reste acyle - de 1 à 50 mol d'oxyde d'éthylène.

5. Procédé selon au moins une des revendications 2 à 4,
**caractérisé en ce qu'**
on effectue l'éthoxylation avec de 5 à 10 mol d'oxyde d'éthylène - rapporté au groupe hydroxyle dans le reste acyle.

6. Procédé selon au moins une des revendications 2 à 5,
**caractérisé en ce qu'**
on met en oeuvre des diamines de formule générale II 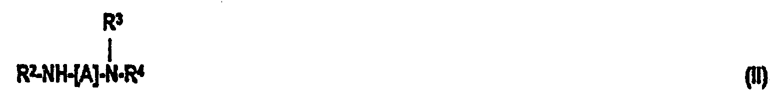 dans laquelle A représente un reste alkylène linéaire ou ramifié ayant de 1 à 6 atomes de carbone, et R², R³ et R⁴ indépendamment l'un de l'autre représente de l'hydrogène ou un groupe alkyle ayant de 1 à 4 atomes de carbone.

7. Procédé selon la revendication 6,
**caractérisé en ce qu'**
on met en oeuvre la diméthyaminopropylamine.

8. Procédé selon au moins une des revendications 2 à 7,
**caractérisé en ce qu'**
on met en oeuvre pour la quaternisation le chlorure de benzyle, le chlorure de méthyle ou le sulfate de diméthyle.

9. Utilisation des composés d'ammonium quaternaires selon la revendication 1, comme agents émulsionnants cationiques en vue de la production de préparations cosmétiques et/ou pharmaceutiques.

10. Utilisation des composés d'ammonium quaternaires selon la revendication1, comme biocides en vue de la préparation de produits de nettoyage et de désinfection.
